# EUROPÄISCHE PATENTSCHRIFT

(11) **EP 2 323 815 B1**
(45) Veröffentlichungstag und Bekanntmachung des Hinweises auf die Patenterteilung: **19.09.2012**
(21) Anmeldenummer: 09777974.8
(22) Anmeldetag: 19.08.2009
(51) Int. Cl.: B25J 9/16

(54) **ROBOTER UND VERFAHREN ZUM STEUERN EINES ROBOTERS**
ROBOT AND METHOD FOR CONTROLLING A ROBOT
ROBOT ET PROCÉDÉ POUR COMMANDER UN ROBOT

(30) Priorität: 27.08.2008 DE 102008041602
(43) Veröffentlichungstag der Anmeldung: 25.05.2011
(73) Patentinhaber: KUKA Laboratories GmbH, 86165 Augsburg (DE)
(72) Erfinder: JACOB, Dirk, 87616 Marktoberdorf (DE); ORTMAIER, Tobias, 30966 Hemmingen (DE)
(74) Vertreter: Patentanwälte Funk & Böss GbR
(86) Internationale Anmeldenummer: PCT/EP2009/006009
(87) Internationale Veröffentlichungsnummer: WO 2010/025828

(56) Entgegenhaltungen:
- WO-A1-2008/004487
- WO-A2-03/044287
- DE-A1- 4 202 505
- JP-A- 58 112 104
- US-A- 5 606 494
- US-A1- 2007 255 454
- US-B1- 6 175 610
- FRAUENHOFER VERBUND MIKROELEKTRONIK: "Berührungslose Bildsteuerung" VME NACHRICHTEN, Dezember 2007 (2007-12), Seiten 4-4, XP002553282 Berlin

## Beschreibung

Die Erfindung betrifft einen Roboter und ein Verfahren zum Steuern eines Roboters.

Roboter sind Handhabungsmaschinen, die zur selbsttätigen Handhabung von Objekten mit zweckdienlichen Werkzeugen ausgerüstet und in mehreren Bewegungsachsen insbesondere hinsichtlich Orientierung, Position und Arbeitsablauf programmierbar sind. Roboter weisen im Wesentlichen einen Roboterarm mit mehreren Achsen und Hebeln auf, die von Antrieben bewegt werden. Die Antriebe sind z.B. elektrische Antriebe.

Der Roboter bzw. dessen Roboterarm kann auch manuell, beispielsweise mittels eines Handgerätes oder durch manuelles Führen bewegt werden. Insbesondere bei einem Bewegen des Roboters durch manuelles Führen, im Rahmen dessen eine Person z.B. am Roboterarm drückt oder zieht, besteht die Gefahr, dass durch ein Berühren des Roboterarms die den Roboter führende Person durch den Roboter verschmutzt wird. Dies ist insbesondere bei einer Verwendung des Roboters im medizinischen Umfeld nachteilig.

Die DE 42 02 505 A1 offenbart eine kontaktlose Führung eines Instruments, welches mittels eines motorisch positionierbaren Stativs bewegt wird. Um das Stativ, welches in bis zu sechs Freiheitsgraden positionierbar ist, kontaktlos zu bewegen, ist eine optische Sendeeinheit vorgesehen, die am Kopf eines Arztes befestigt werden kann. Am Instrument ist eine optische Empfangseinheit angeordnet, sodass das Instrument einer Kopfbewegung des Arztes folgt.

Das Dokument US2009171505 A1 betrifft einen Roboterarm, der mit einem Menschen oder Objekt in Kontakt kommen kann und ein Verfahren zum Steuern dieses Roboterarms. Dabei wird die Bewegung des Menschen bzw. des Objektes berührungslos detektiert, die geometrische Beziehungen zwischen dem Roboterarm und dem Objekt ermittelt und die voraussichtliche Kontaktstelle berechnet. Es werden Ansätze zur Reaktion auf androhende Kollisionen offenbart, wie die Berechnung und Ausführung von Ausweichstrajektorien sowie Einleitung von Impedanzsteuerung für den Roboterarm.

Die Aufgabe der vorliegenden Erfindung ist es, Vorraussetzungen für ein berührungsloses Steuern eines Roboters zu schaffen.

Die Aufgabe der Erfindung wird gelöst durch ein Verfahren zum Steuern eines Roboters, aufweisend folgende Verfahrensschritte:
- Detektieren einer Bewegung eines Objekts mittels in oder an einem Roboterarm eines Roboters und/oder an oder in einem am Roboterarm befestigten Endeffektor angeordneten berührungslosen Abstandssensors und
- aufgrund der mittels des Abstandssensors detektierten Bewegung, Bewegen des Roboterarms derart, um ein Zusammenstoßen des Objekts mit dem Roboterarm zu verhindern und gleichzeitig die Lage einer Befestigungsvorrichtung des Roboterarms oder des Tool Center Points eines am Roboterarm angeordneten Endeffektors konstant zu lassen.

Die Aufgabe der Erfindung wird auch gelöst durch einen Roboter, aufweisend einen Roboterarm mit mehrere Achsen, einer Befestigungsvorrichtung zum Befestigen eines Endeffektors und Antrieben zum Bewegen der Achsen, wenigstens einen am oder im Roboterarm angeordneten berührungslosen Abstandssensor und eine mit den Antrieben und dem Abstandssensor gekoppelte Steuerungsvorrichtung, die eingerichtet ist, den Roboterarm mittels der Antriebe aufgrund einer mittels des Abstandssensors detektierten Bewegung eines Objekts derart zu bewegen, um ein Zusammenstoßen des Objekts mit dem Roboterarm zu verhindern, jedoch die Lage der Befestigungsvorrichtung oder des Tool Center Points des Endeffektors konstant zu lassen.

Alternativ kann der Abstandssensor auch im oder am Endeffektor angeordnet und mit der Steuerungsvorrichtung des erfindungsgemäßen Roboters gekoppelt sein.

Mit den erfindungsgemäßen Robotern kann demnach das erfindungsgemäße Verfahren ausgeführt werden.

Erfindungsgemäß sind demnach im oder am Roboterarm, z.B. an dessen Struktur, oder in oder am Endeffektor der wenigstens eine berührungslose Abstandssensor angeordnet. Berührungslose Abstandssensoren als solche sind allgemein bekannt und erzeugen ein dem Abstand zwischen einem Objekt und dem berührungslosen Abstandssensor zugeordnetes Signal, insbesondere ein elektrisches Signal. Aus diesem Signal ist es dann auch möglich, die Bewegung des Objekts zu ermitteln und/oder die Geschwindigkeit des Objekts relativ zum Roboter aufgrund einer Ableitung des dem Abstand zugeordneten Signals zu bestimmen. Das Objekt kann insbesondere eine z.B. den erfindungsgemäßen Roboter bedienende Person sein, so dass es dieser Person ermöglicht wird, den Roboter berührungslos zu steuern.

Berührungslose Abstandssensoren sind beispielsweise kapazitive Abstandssensoren oder basieren auf anderen physikalischen Gesetzmäßigkeiten.

Erfindungsgemäßen wird aufgrund der detektierten Bewegung der Roboterarm bewegt, um ein Zusammenstoßen des Objekts mit dem Roboterarm zu verhindern und gleichzeitig die Lage einer Befestigungsvorrichtung des Roboterarms oder des Tool Center Points eines am Roboterarm angeordneten Endeffektors konstant zu lassen. Die Steuerungsvorrichtung des erfindungsgemäßen Roboters kann demnach eingerichtet sein, aufgrund der detektierten Bewegung die Achsen über die Antriebe zu bewegen, um das Zusammenstoßen des Objekts mit dem Roboterarm zu verhindern, jedoch die Lage der Befestigungsvorrichtung oder des Tool Center Points des Endeffektors konstant zu lassen. Dies ist z.B. dann möglich, wenn es sich bei dem erfindungsgemäßen Roboter um einen sogenannten redundanten Roboter handelt, so dass dieser eine Bewegung in seinem Nullraum durchführen kann. Als Nullraum wird der Gelenkwinkelraum des redundanten Roboters bezeichnet, in dem eine Umkonfiguration der Robotergelenke derart möglich ist, dass die Lage (Position und Orientierung) des Endeffektors des Roboters im Raum unverändert bleibt. Diese Ausführungsform des erfindungsgemäßen Roboters kann z.B. in der Roboter gestützten Chirurgie verwendet werden, um z.B. einer Person als Objekt, z.B. einem Arzt, Zugang zum Operationsgebiet zu ermöglichen, ohne dafür den erfindungsgemäßen Roboter zu entfernen oder einen mit dem erfindungsgemäßen Roboter durchgeführten Eingriff zu unterbrechen.

Gemäß einer Ausführungsform des erfindungsgemäßen Verfahrens wird der Abstand zwischen dem Objekt und dem Roboter mittels des Abstandsensors detektiert. Aufgrund des mittels des berührungslosen Abstandssensors ermittelten Abstands bzw. dessen Ableitung zwischen dem Objekt und dem Roboter oder der mittels des Abstandssensors ermittelten Bewegung des Objekts kann es beispielsweise vorgesehen sein, z.B. eine Funktion des Roboters und/oder ein Parametrieren der Funktion des Roboters zu initiieren. Die Ableitung des Abstands zwischen dem Objekt und dem Roboter ist die Geschwindigkeit zwischen dem Objekt und dem Roboter. Eine Funktion des Roboters kann z.B. ein Aktivieren und/oder Deaktivieren eines an der Befestigungsvorrichtung angeordneten Endeffektors sein. Dadurch wird es einer den erfindungsgemäßen Roboter bedienenden Person ermöglicht, die Funktion des Endeffektors, der beispielsweise ein mit dem erfindungsgemäßen Roboter geführtes oder bewegtes Werkzeug ist, allgemein berührungslos zu steuern, insbesondere berührungslos ein- und auszuschalten, ohne z.B. ein Bedienfeld zu nutzen. Dies führt nicht nur dazu, dass die Person den erfindungsgemäßen Roboter für ein Aktivieren des Endeffektors nicht zu berühren braucht, sondern kann auch zu einem leichteren Bedienen des erfindungsgemäßen Roboters führen.

Aufgrund des mittels des berührungslosen Abstandssensors ermittelten Abstands bzw. dessen Ableitung zwischen dem Objekt und dem Roboter ermittelt insbesondere die Steuerungsvorrichtung nach einer alternativen Ausführungsform des erfindungsgemäßen Roboters bzw. des erfindungsgemäßen Verfahrens die aufzubringende Soll-Kraft oder das aufzubringende Soll-Drehmoment. Aufgrund der Verwendung des berührungslosen Abstandssensors kann es einer den erfindungsgemäßen Roboter bedienenden Person ermöglicht werden, die vom erfindungsgemäßen Roboter aufzubringende Soll-Kraft bzw. das aufzubringende Soll-Drehmoment in relativ einfacher Weise nicht nur berührungslos, sondern auch relativ einfach intuitiv einzustellen.

Bei der aufzubringenden Soll-Kraft bzw. dem aufzubringenden Soll-Drehmoment handelt es sich nach einer Variante des erfindungsgemäßen Verfahrens bzw. nach einer Variante des erfindungsgemäßen Roboters um eine vom am Roboterarm angeordneten Endeffektor aufzubringende Soll-Kraft bzw. um ein vom am Roboterarm angeordneten Endeffektor aufzubringendes Soll-Drehmoment. Die Steuerungsvorrichtung des erfindungsgemäßen Roboters kann dann derart eingerichtet sein, die Achsen über die Antriebe derart ansteuert, so dass der an der Befestigungsvorrichtung angeordnete Endeffektor die Soll-Kraft oder das Soll-Drehmoment aufbringt.

Ausführungsbeispiele der Erfindung sind exemplarisch in den beigefügten schematischen Zeichnungen dargestellt. Es zeigen:
- Fig. 1: einen Roboter mit berührungslosen Abstandssensoren,
- Figuren 2, 3: ein Erfassungsfeld der berührungslosen Abstandssensoren,
- Figuren 4-6: Flussdiagramme zum Veranschaulichen der Bedienung des Roboters der Fig. 1,
- Fig. 7: einen weiteren Roboter,
- Fig. 8: ein Flussdiagramm zum Veranschaulichen der Bedienung des Roboters der Fig. 7 und
- Fig. 9: einen Endeffektor für einen Roboter.

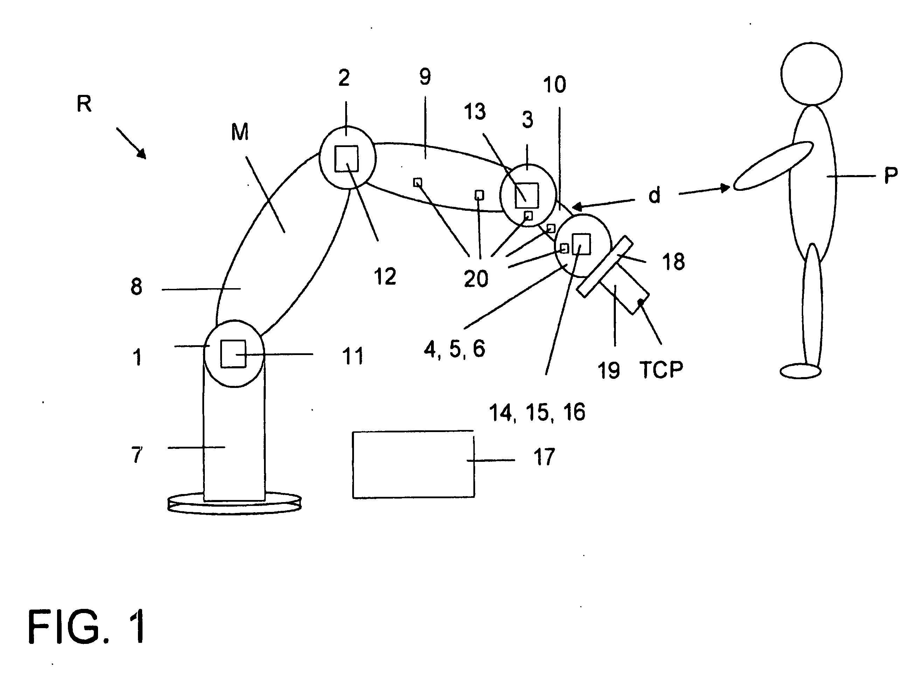

Die Fig. 1 zeigt einen Roboter R mit einem Roboterarm M. Der Roboterarm M stellt im Wesentlichen den beweglichen Teil des Roboters R dar und umfasst mehrere Achsen 1-6, mehrere Hebel 7-10 und einen Flansch 18, an dem ein Endeffektor 19, der z.B. ein medizinisches, insbesondere ein chirurgisches Instrument ist, befestigt ist.

Jede der Achsen 1-6 wird mit einem Antrieb, beispielsweise einem elektrischen Antrieb 11-16 bewegt, die in nicht dargestellter Weise mit einem Steuerrechner 17 des Roboters R elektrisch verbunden sind, so dass der Steuerrechner 17 bzw. ein auf dem Steuerrechner 17 laufendes Rechnerprogramm die elektrischen Antriebe 11-16 derart ansteuern kann, dass die Position bzw. Lage des Flansches 18 des Roboters R bzw. der Tool Center Point TCP des Roboters R im Wesentlichen frei im Raum ausgerichtet werden kann. Die elektrischen Antriebe 11-16 des Roboters R umfassen z.B. jeweils einen elektrischen Motor und gegebenenfalls eine die Motoren ansteuernde Leistungselektronik.

Im Falle des vorliegenden Ausführungsbeispiels weist der Roboter R ferner mit dem Steuerrechner 17 in nicht dargestellter Weise verbundene berührungslose Abstandssensoren 20 auf, die im oder am Roboterarm M angeordnet sind, insbesondere in die Struktur des Roboterarms M integriert oder in einer den Roboterarm M umhüllenden Außenhaut beispielsweise punktuell oder flächenhaft eingelassen sind. Die berührungslosen Abstandssensoren 20 sind z.B., wie es für das vorliegende Ausführungsbeispiel der Fall ist, kapazitive Abstandssensoren 20, die als solche dem Fachmann bekannt sind. Die Abstandssensoren 20 sind eingerichtet, einen Abstand d zu einem Objekt, z.B. zu einer Person P zu ermitteln.

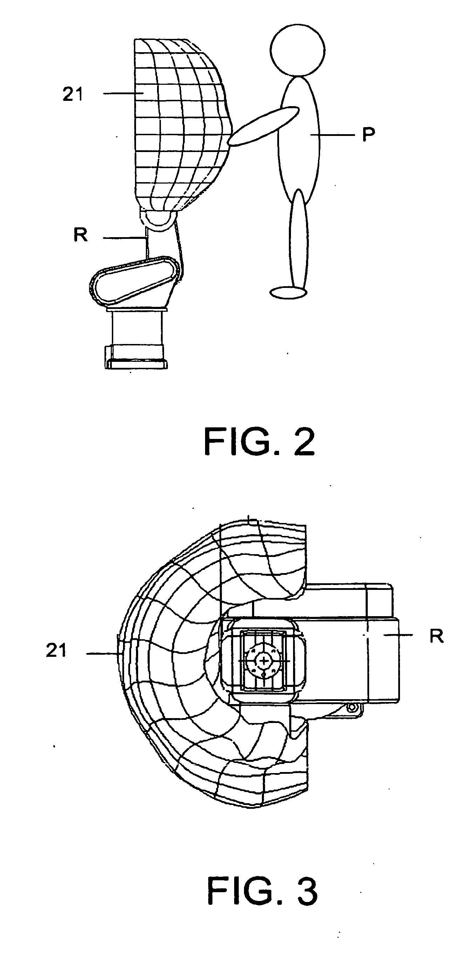

Wie eben erwähnt, handelt es sich bei den berührungslosen Abstandssensoren 20 im Falle des vorliegenden Ausführungsbeispiels um kapazitive Abstandssensoren 20, die zusammen ein den Roboterarm M zumindest teilweise einhüllendes und in den Figuren 2 und 3 dargestelltes Erfassungsfeld 21 erzeugen. Die Fig. 2 zeigt den Roboter R mit dem Erfassungsfeld 21 als Seitenansicht und die Fig. 3 zeigt den Roboter R mit dem Erfassungsfeld 21 als Draufsicht.

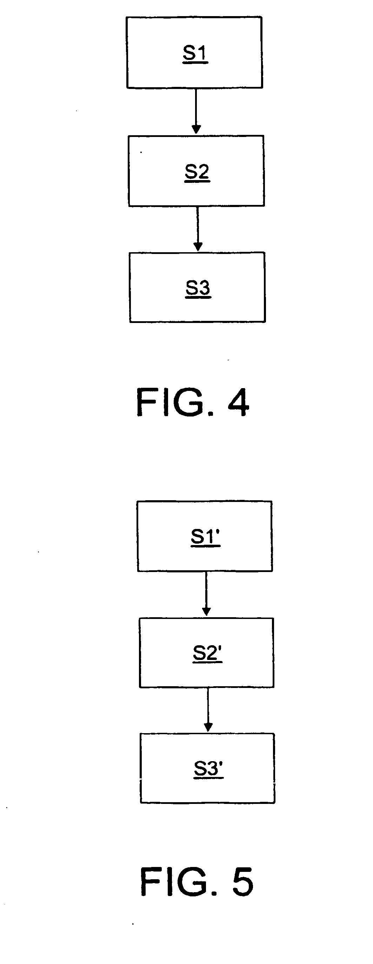

Nähert sich z.B. die Person P diesem Erfassungsfeld 21, dann erzeugen die Abstandssensoren 20 ein dem Abstand d zwischen der Person P und dem Roboter R entsprechendes Signal, das dem Steuerrechner 17 für eine Weiterverarbeitung zugeführt wird. Aufgrund dieses Signals steuert im Falle des vorliegenden Ausführungsbeispiels der Steuerrechner 17 bzw. ein auf dem Steuerrechner 17 laufendes Rechnerprogramm die Bewegung des Roboters R. Dies ist mittels eines in der Fig. 4 dargestellten Flussdiagramms zusammengefasst.

Nähert sich die Person P dem Roboter R, so detektieren die Abstandssensoren 20 den Abstand d zwischen dem Roboter R und der Person P. Die dem Abstand d zugeordneten und von den Abstandssensoren 20 stammenden Signale werden dem Steuerrechner 17 zugeführt, der aufgrund der Signale, insbesondere aufgrund einer erkannten Änderung des Abstands d zwischen der Person P und dem Roboter R eine Bewegung der Person P erkennt, Schritt S1 des Flussdiagramms der Fig. 4.

Aufgrund der detektierten Bewegung der Person P berechnet im Falle des vorliegenden Ausführungsbeispiels der Steuerrechner 17 eine der Bewegung der Person P entsprechende Bewegung, die der Effektor 19 bzw. dessen Tool Center Point TCP ausführen soll, Schritt S2 des Flussdiagramms.

Aufgrund der berechneten Bewegung, die der Effektor 19 bzw. der Tool Center Point TCP ausführen soll, steuert der Steuerrechner 17 die Antriebe 11-16 derart an, so dass der Effektor 19 bzw. der Tool Center Point TCP diese Bewegung ausführt ohne dass die Person P den Roboter R berührt, Schritt S3 des Flussdiagramms der Fig. 4.

In einem weiteren Ausführungsbeispiel ist der Roboter R bzw. der Steuerrechner 17 derart konfiguriert, so dass mittels der Abstandssensoren 20 eine vom Roboter R aufzubringende Soll-Kraft oder Soll-Drehmoment und/oder deren jeweilige Ableitungen berechnet werden. Dies ist mittels eines in der Fig. 5 dargestellten Flussdiagramms zusammengefasst.

Nähert sich die Person P dem Roboter R, so detektieren die Abstandssensoren 20 den Abstand d zwischen dem Roboter R und der Person P. Die dem Abstand d zugeordneten und von den Abstandssensoren 20 stammenden Signale werden dem Steuerrechner 17 zugeführt, Schritt S1' des Flussdiagramms der Fig. 5.

Aufgrund des detektierten Abstands d berechnet im Falle des vorliegenden Ausführungsbeispiels der Steuerrechner 17 eine dem Abstand d entsprechende Soll-Kraft oder Soll-Drehmoment und/oder deren jeweilige Ableitungen, die der am Roboterarm M angeordnete Endeffektor 19 aufbringen soll, Schritt S2' des Flussdiagramms der Fig. 5.

Anschließend steuert der Steuerrechner 17 die Antriebe 11-16 derart an, so dass der Endeffektor 19 die berechnete Soll-Kraft auf ein in den Figuren nicht näher dargestelltes Objekt aufbringt, Schritt S3' des Flussdiagramms der Fig. 5. Somit ist es möglich, die vom Endeffektor 19 aufzubringende Kraft und/oder deren Ableitung berührungslos über den Abstand d zum Roboter R einzustellen. Alternativ ist es auch möglich, anstelle einer Kraft ein vom Endeffektor 19 aufzubringendes Drehmoment und/oder deren Ableitung über den Abstand d zwischen der Person P und dem Roboter R einzustellen.

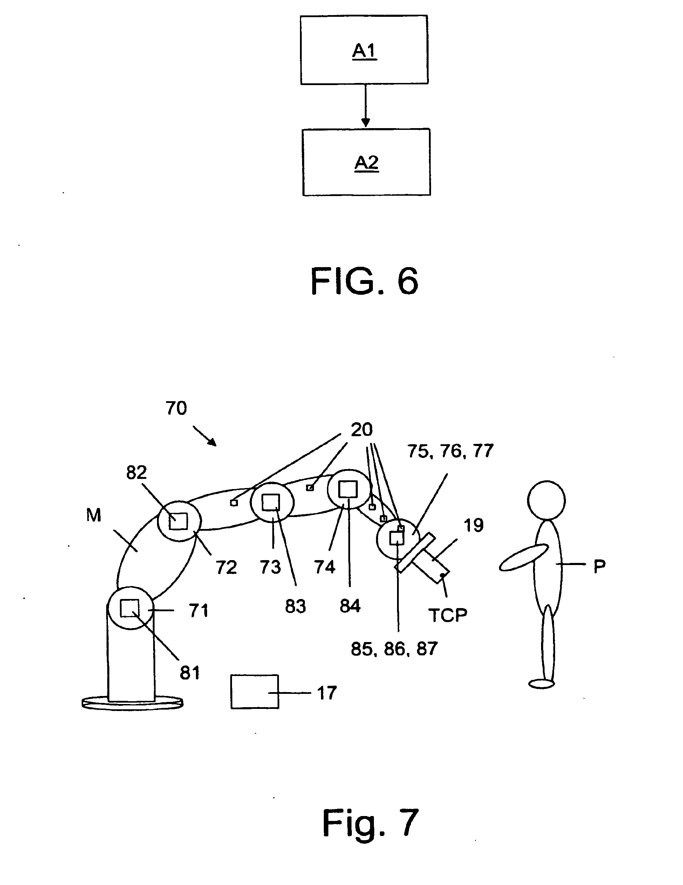

In einem weiteren Ausführungsbeispiel ist der Roboter R bzw. der Steuerrechner 17 derart konfiguriert, so dass eine Funktion des Roboters R und/oder ein Parametrieren einer Funktion des Roboters R automatisch ausgeführt wird, wenn der Abstand d zwischen der Person P und dem Roboter R einen Mindestabstand unterschreitet oder einen Maximalabstand überschreitet. Dies ist mittels eines in der Fig. 6 dargestellten Flussdiagramms zusammengefasst.

Nähert sich die Person P dem Roboter R derart, so dass der Abstand d den Mindestabstand unterschreitet, so wird dies von den Abstandssensoren 20 bzw. vom Steuerrechner 17 erkannt, Schritt A1 des Flussdiagramms der Fig. 6.

Daraufhin aktiviert im Falle des vorliegenden Ausführungsbeispiels der Steuerrechner 17 den Endeffektor 19, Schritt A2 des Flussdiagramms der Fig. 6.

Die Fig. 7 zeigt einen weiteren Roboter 70 mit einem mehrere Achsen aufweisenden Roboterarm M. Bei dem Roboter 70 handelt es sich im Falle des vorliegenden Ausführungsbeispiels um einen redundanten Roboter R, weshalb dieser eine Bewegung in seinem Nullraum durchführen kann. Als Nullraum wird der Gelenkwinkelraum des redundanten Roboters 70 bezeichnet, in dem eine Umkonfiguration der Robotergelenke 71-77 derart möglich ist, dass die Lage (Position und Orientierung) des Endeffektors 19 im Raum unverändert bleibt. Diese Ausführungsform des Roboters 70 kann z.B. in der Roboter gestützten Chirurgie verwendet werden.

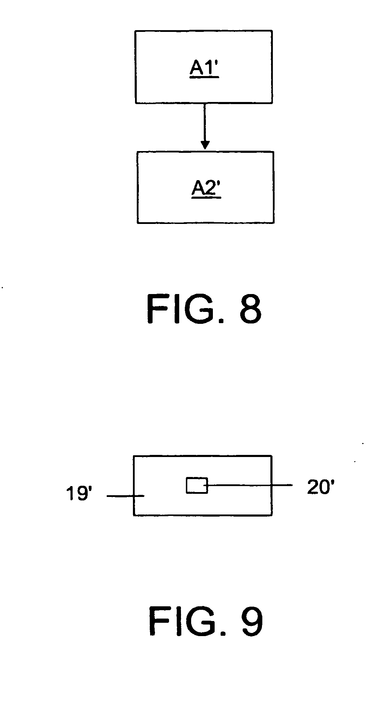

Im Falle des vorliegenden Ausführungsbeispiels kann die Person P den Roboter 70 folgendermaßen bedienen, was mittels eines in der Fig. 8 dargestellten Flussdiagramms veranschaulicht ist.

Nähert sich die Person P dem Roboter 70, so wird dies mittels der Abstandssensoren 20 erkannt, Schritt A1' des Flussdiagramms der Fig. 8, indem z.B. das dem Abstand zugeordnete Signal abgeleitet wird.

Um ein Zusammenstoßen der Person P mit dem relevanten Teil des Roboters 70 zu vermeiden, steuert der Steuerrechner 17 aufgrund der erkannten Annäherung der Person P an den Roboter 70 die Antriebe 81-87 des Roboters 70 derart an, dass einerseits die Lage des Endeffektors 19 im Raum unverändert bleibt, der relevante Hebel bzw. das relevante Gelenk 71-77, mit dem die Person P potenziell zusammenstößt, dem Annähern der Person P weicht, Schritt A2' des Flussdiagramms der Fig. 8.

Die obenstehend beschriebenen Roboter R, 70 umfassen die Abstandssensoren 20, die in bzw. an den entsprechenden Roboterarmen M angeordnet sind. Es ist aber auch möglich, zusätzlich oder alternativ die Abstandssensoren 20 am Endeffektor anzuordnen. Die Fig. 9 zeigt einen solchen Endeffktor 19', in oder an dem Abstandssensoren 20' angeordnet sind.

## Patentansprüche

1. Verfahren zum Steuern eines Roboters, aufweisend
- Detektieren einer Bewegung eines Objekts (P) mittels in oder an einem Roboterarm (M) eines Roboters (R, 70) und/oder an oder in einem am Roboterarm (M) befestigten Endeffektor (19') angeordneten berührungslosen Abstandssensors (20, 20'), und
- aufgrund der mittels des Abstandssensors (20, 20') detektierten Bewegung, Bewegen des Roboterarms (M) derart, um ein Zusammenstoßen des Objekts (P) mit dem Roboterarm (M) zu verhindern und gleichzeitig die Lage einer Befestigungsvorrichtung (18) des Roboterarms (M) oder des Tool Center Points (TCP) eines am Roboterarm (M) angeordneten Endeffektors (19, 19') konstant zu lassen.

2. Verfahren nach Anspruch 1, aufweisend Auslösen einer Funktion des Roboters (R) und/oder ein Parametrieren einer Funktion des Roboters (R) aufgrund der Bewegung.

3. Verfahren nach Anspruch 1, aufweisend
- Detektieren des Abstands (d) zwischen dem Objekt (P) und dem Roboter (R, 70) mittels des Abstandsensors (20, 20') und
- Ermitteln einer vom Roboter (R) aufzubringenden Soll-Kraft oder Soll-Drehmoments aufgrund des detektierten Abstands (d) und/oder aufgrund einer Ableitung des detektierten Abstands (d) zwischen dem Objekt (P) und dem Roboter (R), oder Auslösen einer Funktion des Roboters (R) und/oder ein Parametrieren einer Funktion des Roboters (R) aufgrund eines mittels des Abstandsensors (20, 20') detektierten Abstands und/oder dessen Ableitung zwischen dem Objekt (P) und dem Roboter (R).

4. Verfahren nach Anspruch 2 oder 3, aufweisend Ansteuern des Roboters (R) derart, so dass ein am Roboterarm (M) angeordneter Endeffektor (19, 19') die ermittelte Soll-Kraft und/oder das ermittelte Soll-Drehmoment aufbringt.

5. Verfahren nach einem der Ansprüche 2 bis 4, bei dem die Funktion des Roboters (R) ein Aktivieren und/oder Deaktivieren eines an der Befestigungsvorrichtung (18) angeordneten Endeffektors (19, 19') ist.

6. Verfahren nach einem der Ansprüche 1 bis 5, bei dem der Abstandssensor ein kapazitiver Abstandssensor (20, 20') ist.

7. Roboter, aufweisend
- einen Roboterarm (M) mit mehrere Achsen (1-6, 71-77), einer Befestigungsvorrichtung (18) zum Befestigen eines Endeffektors (19, 19') und Antrieben (11-16, 81-87) zum Bewegen der Achsen (1-6),
- wenigstens einen am oder im Roboterarm (M) oder im oder am Endeffektor (19') angeordneten berührungslosen Abstandssensor (20, 20') und
- eine mit den Antrieben (11-16, 81-87) und dem Abstandssensor (20, 20') gekoppelte Steuerungsvorrichtung (17), die eingerichtet ist, den Roboterarm (M) mittels der Antriebe (11-16, 81-87) aufgrund einer mittels des Abstandssensors (20) detektierten Bewegung eines Objekts (P) derart zu bewegen, um ein Zusammenstoßen des Objekts (P) mit dem Roboterarm (M) zu verhindern, jedoch die Lage der Befestigungsvorrichtung (18) oder des Tool Center Points (TCP) des Endeffektors (19, 19') konstant zu lassen.

8. Roboter nach Anspruch 7, dessen Steuerungsvorrichtung (17) eingerichtet ist,
- aufgrund eines mittels des Abstandssensors (20, 20') detektierten Abstands (d) zwischen dem Objekt (P) und dem Roboter (R) und/oder dessen Ableitung eine von dem Roboter (R) aufzubringende Soll-Kraft oder Soll-Drehmoment zu ermitteln, und/ oder
- aufgrund der mittels des Abstandssensors (20, 20') detektierten Bewegung und/oder aufgrund eines detektierten Abstands und/oder deren Ableitung eine Funktion des Roboters (R) auszulösen und/oder eine Funktion des Roboters (R) zu parametrieren.

9. Roboter nach Anspruch 8, bei dem
- die Funktion des Roboters (R) ein Aktivieren und/oder Deaktivieren eines an der Befestigungsvorrichtung (18) angeordneten Endeffektors (19, 19') ist, und/oder
- die Steuerungsvorrichtung (17) die Achsen (1-6, 71-77) über die Antriebe (11-16, 81-87) derart ansteuert, so dass der an der Befestigungsvorrichtung angeordnete Endeffektor (19, 19') die Soll-Kraft oder das Soll-Drehmoment aufbringt.

10. Roboter nach eine der Ansprüche 7 bis 9, bei dem der Abstandssensor ein kapazitiver Abstandssensor (20, 20') ist.

## Claims

1. Method for controlling a robot, comprising:
- detecting a movement of an object (P) by means of a non-contact distance sensor (20, 20') arranged in or on a robot arm (M) of a robot (R, 70) and/or on or in an end effector (19') secured to the robot arm (M), and
- moving the robot arm (M) on the basis of the movement detected by means of the distance sensor (20, 20') in order to prevent a collision of the object (P) with the robot arm (M) and at the same time to keep constant the position of a securing device (18) of the robot arm (M) or the tool center point (TCP) of an end effector (19, 19') arranged on the robot arm (M).

2. Method according to claim 1, comprising triggering a function of the robot (R) and/or parameterizing a function of the robot (R) on the basis of the movement.

3. Method according to claim 1, comprising
- detecting the distance (d) between the object (P) and the robot (R, 70) by means of the distance sensor (20, 20') and
- determining a reference force or reference torque to be applied by the robot (R) on the basis of the detected distance (d) and/or on the basis of a derivation of the detected distance (d) between the object (P) and the robot (R), or triggering a function of the robot (R) and/or parameterizing a function of the robot (R) on the basis of a distance detected by the distance sensor (20, 20') and/or its derivation between the object (P) and the robot (R).

4. Method according to claim 2 or 3 comprising controlling the robot (R) such that an end effector (19, 19') arranged on the robot arm (M) applies the determined reference force and/or the determined reference torque.

5. Method according to any one of claims 2 to 4, in which the function of the robot (R) is activating and/or deactivating an end effector (19, 19') arranged on the securing device (18).

6. Method according to any one of claims 1 to 5, in which the distance sensor is a capacitive distance sensor (20, 20').

7. Robot comprising
- a robot arm (M) with several axes (1-6, 71-77), a securing device (18) for securing an end effector (19, 19') and drives (11-16, 81-87) for moving the axes (1-6),
- at least one non-contact distance sensor (20, 20') arranged on or in the robot arm (M) or in or on the end effector (19') and
- a control device (17) coupled to the drives (11-16, 81-87) and the distance sensor (20, 20'), which is set up to move the robot arm (M) by means of the drives (11-16, 81-87) on the basis of a movement of an object (P) detected by the distance sensor (20) in order to prevent the collision of the object (P) with the robot arm (M), and still keep constant the position of the securing device (18) or the tool center point (TCP) of the end effector (19, 19').

8. Robot according to claim 7, the control device (17) of which is set up
- to determine, on the basis of a distance (d) detected by means of the distance sensor (20, 20') between the object (P) and the robot (R) and/or its derivation, a reference force or reference torque to be applied by the robot (R) and/or
- to trigger on the basis of a movement detected by means of the distance sensor (20,20') and/or on the basis of a detected distance and/or their derivation a function of the robot (R) and/or to parameterize a function of the robot (R).

9. Robot according to claim 8, in which
- the function of the robot (R) is to activate and/or deactivate an end effector (19, 19') arranged on the securing device (18) and/or
- the control device (17) controls the axes (1-6, 71-77) by means of the drives (11-16, 81-87), such that the end effector (19, 19') arranged on the securing device applies the reference force or the reference torque.

10. Robot according to any one of claims 7 to 9 in which the distance senor is a capacitive distance sensor (20, 20').

## Revendications

1. Procédé de commande d'un robot, présentant:
- la détection d'un mouvement d'un objet (P) au moyen d'un capteur de distance (20, 20') sans contact agencé dans ou sur un bras de robot (M) d'un robot (R, 70) et/ou sur ou dans un effecteur terminal (19') fixé sur le bras de robot (M), et
- sur la base du déplacement détecté au moyen du capteur de distance (20, 20'), déplacement du bras de robot (M) de manière à éviter une collision de l'objet (P) avec le bras de robot (M) et, simultanément, à maintenir constante la position d'un dispositif de fixation (18) du bras de robot (M) ou du point centre outil (TCP) d'un effecteur terminal (19, 19') agencé sur le bras de robot (M).

2. Procédé selon la revendication 1 présentant le déclenchement d'une fonction du robot (R) et/ou un paramètrage d'une fonction du robot (R) sur la base du déplacement.

3. Procédé selon la revendication 1 présentant
- la détection de la distance (d) entre l'objet (P) et le robot (R, 70) au moyen du capteur de distance (20, 20') et
- la détermination d'une force de consigne ou d'un couple de consigne à appliquer par le robot (R) sur la base de la distance (d) détectée et/ou sur la base d'une dérivation de la distance (d) détectée entre l'objet (P) et le robot (R), ou le déclenchement d'une fonction du robot (R) et/ou un paramétrage d'une fonction du robot (R) sur la base d'une distance détectée au moyen du capteur de distance (20, 20') et/ou de sa dérivation entre l'objet (P) et le robot (R).

4. Procédé selon la revendication 2 ou 3, présentant le pilotage du robot (R) de telle sorte qu'un effecteur terminal (19, 19') agencé sur le bras de robot (M) applique la force de consigne déterminée et/ou le couple de consigne déterminé.

5. Procédé selon l'une des revendications 2 à 4, dans lequel la fonction du robot (R) est une activation et/ou une désactivation d'un effecteur terminal (19, 19') agencé sur le dispositif de fixation (18).

6. Procédé selon l'une des revendications 1 à 5, dans lequel le capteur de distance est un capteur de distance (20, 20') capacitif.

7. Robot présentant
- un bras de robot (M) comportant plusieurs axes (1-6, 71-77) d'un dispositif de fixation (18) pour la fixation d'un effecteur terminal (19, 19') et des entraînements (11-16, 81-87) pour le déplacement des axes (1-6).
- au moins un capteur de distance (20, 20') sans contact agencé sur ou dans le bras de robot (M) ou dans ou sur l'effecteur terminal (19'), et
- un dispositif de commande (17) couplé aux entraînements (11-16, 81-87) et au capteur de distance (20), qui est équipé pour déplacer le bras de robot (M) au moyen des entraînements (11-16, 81-87) sur la base d'un mouvement d'un objet (P), détecté au moyen du capteur de distance (20), de manière à éviter une collision de l'objet (P) avec le bras de robot (M) et, tout en maintenant cependant constante la position du dispositif de fixation (18) ou du point centre outil (TCP) de l'effecteur terminal (19, 19').

8. Robot selon la revendication 7, dont le dispositif de commande (17) est équipé pour
- sur la base d'une distance (d) détectée au moyen du capteur de distance (20, 20') entre l'objet (P) et le robot (R) et/ou sur la base d'une dérivation de la distance (d), déterminer une force de consigne ou un couple de consigne à appliquer par le robot (R) et/ou
- sur la base du déplacement détecté au moyen du capteur de distance (20) et/ou sur la base d'une distance détectée et/ou sur la base de sa dérivation, déclencher une fonction du robot (R) et/ou paramétrer une fonction du robot (R).

9. Robot selon la revendication 8, dans lequel
- la fonction du robot (R) est une activation et/ou une désactivation d'un effecteur terminal (19, 19') agencé sur le dispositif de fixation (18), et/ou
- le dispositif de commande (17) pilote les axes (1-6, 71-77) via les entraînements (11-16, 81-87) de telle sorte que l'effecteur terminal (19, 19') agencé sur le dispositif de fixation applique la force de consigne ou le couple de consigne.

10. Robot selon l'une des revendications 7 à 9, dans lequel le capteur de distance est un capteur de distance (20, 20') capacitif.
